# EUROPÄISCHE PATENTSCHRIFT

(11) **EP 0 975 970 B1**
(45) Veröffentlichungstag und Bekanntmachung des Hinweises auf die Patenterteilung: **11.12.2002**
(21) Anmeldenummer: 99903627.0
(22) Anmeldetag: 13.01.1999
(51) Int. Cl.: G01N 33/564, G01N 33/76, G01N 33/543, C12N 15/00

(54) **REZEPTORBINDUNGSASSAYS UND REAGENZIENSATZ ZUM NACHWEIS VON TSH-REZEPTOR-AUTOANTIKÖRPERN**
RECEPTOR BINDING ASSAYS AND REAGENT KIT FOR DETECTING TSH RECEPTOR AUTO-ANTIBODIES
TESTS DE LIAISON AU RECEPTEUR ET KIT DE REACTIFS POUR LA DETECTION D'AUTO-ANTICORPS A RECEPTEUR TSH

(30) Priorität: 15.01.1998 DE 19801319
(43) Veröffentlichungstag der Anmeldung: 02.02.2000
(73) Patentinhaber: B.R.A.H.M.S Aktiengesellschaft, 16761 Hennigsdorf (DE)
(72) Erfinder: BERGMANN, Andreas, D-12351 Berlin (DE); STRUCK, Joachim, D-12161 Berlin (DE); MORGENTHALER, Nils, D-12099 Berlin (DE); WEGLÖHNER, Wolfgang, D-12099 Berlin (DE); HOLLIDT, Jörg-Michael, D-12165 Berlin (DE)
(74) Vertreter: Andrae, Steffen, Dr.
(86) Internationale Anmeldenummer: EP9900159
(87) Internationale Veröffentlichungsnummer: WO99036782

(56) Entgegenhaltungen:
- WO-A-95/06258
- WO-A-98/26294
- CHEMICAL ABSTRACTS, vol. 127, no. 1, 5. Januar 1998 Columbus, Ohio, US; abstract no. 679865, XP002103277 & W.B. MINICH ET AL.: "Expression of a functional tagged human thyrotropin receptor in hela cells using recombinant vaccinia virus" EXPERIMENTAL AND CLINICAL ENDOCRINOLOGY AND DIABETES, Bd. 105, Nr. 5, 1997, Seiten 282-290, New York NY USA

## Beschreibung

Die vorliegende Erfindung betrifft verbesserte Rezeptorbindungsassays zur Bestimmung von TSH-Rezeptor-Autoantikörpern (TSHR-Auto-Ab), die bei Schilddrüsen-Autoimmunerkrankungen, insbesondere beim Morbus Basedow, auftreten.

Es ist bekannt, daß zahlreiche Erkrankungen, an denen die Schilddrüse beteiligt ist, Autoimmunerkrankungen sind, bei denen Autoantikörper gegen molekulare Strukturen der Schilddrüse gebildet werden, die im Zusammenhang mit der Erkrankung beginnen, als Autoantigene zu wirken. Die wichtigsten bekannten Auotantigene der Schilddrüse sind dabei Thyreoglobulin (Tg), die Schilddrüsenperoxidase (TPO) und insbesondere der TSH-Rezeptor (TSHR) (vgl. Furmaniak J et al., Autoimmunity 1990, Vol. 7, S. 63-80).

Der TSH-Rezeptor ist ein in der Schilddrüsenmembran lokalisierter Rezeptor, an den das von der Hypophyse ausgeschüttete Hormon TSH (Thyroid-stimulierendes Hormon oder Thyreotropin) bindet und dadurch die Ausschüttung der eigentlichen Schilddrüsenhormone, insbesondere des Thyroxins, auslöst. Der TSH-Rezeptor gehört zur Rezeptor-Familie der G-Protein-gekoppelten Glykoprotein-Rezeptoren mit einer großen aminoterminalen extrazellulären Domäne, zu der auch der LH/CG- und der FSH-Rezeptor gehören. Eine Aufklärung der chemischen Struktur des TSH-Rezeptors, d.h. der Sequenz der für ihn codierenden DNA sowie der daraus ableitbaren Aminosäuresequenz des Rezeptors selbst, gelang Ende 1989 (vgl. Libert F. et al., Biochem. Biophys. Res. Commun. 165: 1250-1255; Nagayama Y. et al., Biochem. Biophys. Res. Commun. 165: 1184-1190; vgl. auch EP-A-0433509 bzw. WO-A-91/09121; sowie WO-A-91/09137; WO-A-91/10735 und WO-A-91/03483; ferner Yuji Nagayama & Basil Rapoport, in: Molecular Endocrinology, Vol. 6 No. 2, S. 145-156 und die darin zitierte Literatur).

Es ist allgemein bekannt, daß bei der als Morbus Basedow (englisch: Graves disease) bekannten Schilddrüsen-Autoimmunerkrankung stimulierende Autoantikörper eine Rolle spielen, die gegen der TSH-Rezeptor gebildet werden und mit diesem so wechselwirken, daß die Schilddrüse stimuliert wird, was sich als Schilddrusenüberfunktion (Hyperthyreose) äußert. Die Bestimmung von Autoantikörpern gegen den TSH-Rezeptor hat somit für die Diagnose des Morbus Basedow eine erhebliche klinische Bedeutung.

TSHR-Auto-Ab werden in biologischen Proben bisher im wesentlichen nach zwei Verfahrensprinzipien bestimmt (vgl. z.B Morgenthaler N.G. at al., J Clin Endocrinol Metab 81: 3155-3161 (1996)):

Bei Zellstimulationstests äußert sich die Anwesenheit von stimulierenden TSHR-Auto-Ab, die in der Literatur häufig mit der Abkürzung TSI (TSI = thyroid stimulating immunoglobulins) bezeichnet werden, dadurch, daß bestimmte Funktionen von geeigneten Zellen, die in ihrer Zellmembran natürliche oder rekombinante TSHR aufweisen und mit einer Autoantikörper enthaltenden Probe in Kontakt kommen, durch Stimulation ausgelöst oder verstärkt werden, insbesondere die Bildung von cAMP (cyclischem Adenosinmonophosphat). Bei diesen auch als Bioassays bezeichneten Tests wird selektiv die stimulierende Wirkung gemessen, die Messung ist jedoch außerordentlich aufwendig und daher für die klinische Routinediagnostik wenig geeignet.

Alternativ dazu können Autoantikörper auch unter Verwendung von kompetitiven Rezeptorbindungsassays, insbesondere Radiorezeptorassays, bestimmt werden, z.B. unter Verwendung des TRAK-Assay® der B.R.A.H.M.S Diagnostica GmbH. Zur Bestimmung von TSH-Rezeptor-Autoantikörpern (TSHR-Auto-Ab) wird nach diesem herkömmlichen Verfahren so vorgegangen, daß man die zu bestimmenden Autoantikörper aus einer Serumprobe in flüssiger Phase mit einem radioaktiv markierten bovinen TSH (¹²⁵I-bTSH) um die Bindungsstellen eines solubilisierten porcinen TSH-Rezeptors (porc.TSHR) konkurrieren läßt (vgl. Southgate, K. et al., Clin. Endocrinol. (Oxford) 20, 539-541 (1984); Matsuba T. et al., J.Biochem.118, S.265-270 (1995); EP 719 858 A2; Produktinformation zum TRAK-Assay® der Firma B.R.A.H.M.S Diagnostica GmbH). Um das an die porc.TSHR-Präparation gebundene ¹²⁵I-bTSH zu bestimmen, wird nach Abschluß der Inkubation der eingesetzte solubilisierte porc.TSHR mit einem Fällungsreagens und einem anschließenden Zentrifugierschritt von der flüssigen Phase abgetrennt. Die Bestimmung des Rezeptor-gebundenen ¹²⁵I-bTSH erfolgt durch Messung der im Sediment gebundenen Radioaktivität. Da die Bestimmung auf einer Konkurrenz (Kompetition) zwischen ¹²⁵I-bTSH und den zu bestimmenden Autoantikörpern um gemeinsame Bindungsstellen auf dem porc. TSHR beruht, werden bei diesem Verfahren alle solchen und nur solche Autoantikörper erfaßt, die tatsächlich mit bTSH kompetieren. Solche kompetierenden, zur Inhibierung der TSH-Bindung befähigten Autoantikörper werden in der Literatur auch als TBII (TBII = thyrotropin-binding inhibitory immunoglobulin) bezeichnet, und das Ausmaß ihrer Aktivität wird auch als prozentuale sogenannte TBII-Aktivität angegeben.

Die bisher bekannten kompetitiven Radiorezeptorassays zum Nachweis von TSH-Rezeptor-Autoantikörpern weisen verschiedene Nachteile auf, die auf die Qualität bzw. Verfügbarkeit der verwendeten Assaykomponenten, in den Seren einzelner Patienten vorkommende Anomalitäten, die in den bekannten Assays die Meßergebnisse verfälschen können, sowie darauf zurückzuführen sind, daß die Bindungsfähigkeit von TSH-Rezeptorpräparationen generell sehr empfindlich auf Veränderungen des Rezeptors oder der von ihm gebundenen Biomoleküle reagiert. Die Bindung von Biomolekülen von peptidischer oder proteinischer Natur, z.B. von Hormonen oder auch Autoantikörpern, an Rezeptoren ist in der Regel sehr komplexer Natur, und die Ausbildung einer spezifischen Bindung zwischen Rezeptor und Biomolekül ist sehr viel empfindlicher gegenüber strukturellen Veränderungen insbesondere des Rezeptors, als das bei einem üblichen Bindungspaar Antigen/Antikörper der Fall ist, das Grundlage der meisten Immunoassays ist, bei denen Rezeptoren keine Rolle spielen. Versuche, den TSH-Rezeptor zu immobilisieren und/oder zu markieren, führten bisher in der Regel zu strukturellen Veränderungen, die die Funktionalität des Rezeptors stark beeinträchtigen. Das hat zur Folge, daß bisher kaum nacharbeitbare Beschreibungen einer praktischen Umsetzung zahlreicher Assay-Grundtypen, die bei Immunoassays unter Ausnutzung einer Antikörper/Antigen-Bindung zur Verfügung stehen, für den Fall von Rezeptorbindungsassays zur Bestimmung von TSHR-Auto-Ab veröffentlicht wurden, so daß derartige andere Assaytypen in der Praxis für die TSHR-Auto-Ab-Bestimmung noch nicht genutzt werden können. Das gilt insbesondere für solche Assaytypen, bei, denen mit immobilisierten Bindungspartnern gearbeitet wird und am Ende der Messung direkt die Konzentration einer an eine Festphase gebundenen Markierung bestimmt wird oder bei denen zur Markierung voluminöse Moleküle wie Enzyme, Enzymsubstrate oder Chemilumineszenzlabel verwendet werden. Da die Messung einer an eine Festphase gebundenen Markierung Grundlage der meisten Assay-Automaten für Reihenmessungen ist, sind die bekannten Assays zur Bestimmung von TSH-Rezeptor-Autoantikörpern bisher nicht auf derartigen Automaten durchführbar.

In dem Patent DE 43 28 070 C1 ist ein Typ eines Rezeptorbindungsassays, der nach der Coated-Tube-Technik arbeitet, beschrieben, bei dem die Schwierigkeit der Herstellung von markierten bzw. immobilisierten funktionalen Rezeptorpräparationen dadurch umgangen wird, daß, man an die Festphase Bestandteile eines kompetitierenden Reaktionsystems bindet, das gewissermaßen einen "Schatten" der eigentlichen Rezeptorbindungsreaktion darstellt. Für die Schaffung von Assays für die klinische Routinediagnostik hat sich das offenbarte Verfahrensprinzip jedoch als zu kompliziert und daher wenig praktikabel erwiesen. Auf die allgemeinen Ausführungen in der genannten Patentschrift zur Problematik von Rezeptorbindungsassays im allgemeinen und von solchen zur Bestimmung von TSH-Rezeptor-Autoantikörpern im speziellen wird ergänzend ausdrücklich Bezug genommen.

Aus der EP-B-0 488 170 sind zellfreie Rezeptorbindungstests bekannt, bei denen rekombinante Fusionsrezeptoren aus einem aminoterminalen Rezeptorprotein und einem Trägerprotein, insbesondere dem konstanten Teil (Fc) der schweren Kette eines Immunglobulins, eingesetzt werden, die mittels eines Antiserums oder eines monoklonalen Antikörpers an eine feste Phase gekoppelt sind. Die diskutierten Rezeptoren gehören nicht zur Klasse der hochmolekularen G-Protein gekoppelten Glykoprotein-Rezeptoren. Ferner ist eine Immobilisierung durch Bindung eines Trägerproteins, das der Fc-Teil eines Immunglobulins ist, für Rezeptorbindungsassays, mit deren Hilfe Autoantikörper bestimmt werden sollen, wenig geeignet, da die Autoantikörper selbst zu den Immunglobulinen gehören und an das Immobilisierungssystem binden können.

Bestimmte Nachteile der herkömmlichen Assays haben auch damit zu tun, daß zur Bestimmung humaner TSHR-Auto-Ab Reaktionspartner unterschiedlicher tierischer Herkunft, d.h. solubilisierte porcine TSHR-Präparationen in Verbindung mit markiertem bTSH, verwendet wurden. Die genannten Assaykomponenten zeichnen sich zwar durch eine gute gegenseitige Bindung aus und ermöglichen eine Erfassung z.B. von etwa 80% bis 90% der bei Morbus Basedow vorkommenden humanen TSHR-Auto-Ab. Die gegenüber einer 100%igen Erfassung der nachzuweisenden TSHR-Auto-Ab verminderte klinische Wertigkeit ist jedoch vermutlich mindestens teilweise auch darauf zurückzuführen, daß die in Patientenseren vorkommenden Autoantikörper gegen den humanen TSHR gerichtet sind, jedoch aufgrund ihrer Bindung an eine porcine TSHR-Präparation bestimmt werden. Totz der grundsätzlichen Verfügbarkeit von rekombinant erzeugten humanen TSHR-Präparationen (rhTSHR) wurden derartige rhTSHR in klinischen Assays noch nicht eingesetzt, da evtl. zu erwartende Vorteile durch zahlreiche neue praktische Nachteile entwertet wurden. Insbesondere war es bisher genausowenig wie im Falle des porc.TSHR möglich, einen funktionalen, nativen rhTSHR in Assays in festphasengebundener (immobilisierter) oder markierter Form einzusetzen.

Aus den Veröffentlichungen W.B.Minich, M.Behr, und U.Loos, Exp Clin Endocrinol Diabetes 105, 282-290 (1997) sowie "70th Annual Meeting of the American Thyroid Association", 15.-19.10.1997, Abstract No. 89 bzw. W.B. Minich, J.D.Weymayer, U.Loos, Thyroid, Vol.8, 3-7 (1998) (im Druck) ist es bekannt, daß es möglich ist, einen rekombinanten humanen Fusions-TSHR über einen gentechnologisch angefügten Peptidrest zu immobilisieren und in dieser Form in TSHR-Auto-Ab-Bestimmungen zu verwenden. Eine entsprechende zusammenfassende Offenbarung findet sich auch in der nicht vorveröffentlichten Internationalen Patentanmeldung WO 98/20343.

In der EP-A 0 719 858 wird ferner ein Verfahren zur Herstellung eines funktionalen rhTSHR mit Hilfe einer Myelom-Zellinie beschrieben. Die Anmeldung erwähnt in allgemeiner, spekulativer Form die Möglichkeit, unter Verwendung des hergestellten rhTSHR-Polypeptids monoklonale Antikörper zu erzeugen, und macht den Vorschlag, solche Antikörper u.a. zur Immobilisierung des rhTSHR zu verwenden und davon bei der Bestimmung von TSHR-Auto-Ab Gebrauch zu machen. Es wird jedoch weder die tatsächliche Herstellung und Selektion derartiger monoklonaler Antikörper beschrieben, noch wird konkret gezeigt, daß es dem Vorschlag entsprechend tatsächlich möglich ist, mit den ggf. erhältlichen monoklonalen Antikörpern einen rhTSHR ohne Funktiönalitätsverlust zu immobilisieren und in dieser Form bei der Bestimmung von TSHR-Auto-Ab zu nutzen. Die Offenbarung von EP-A 0 719 858 ist insoweit nicht nacharbeitbar. In der zugrunde liegenden wissenschaftlichen Veröffentlichung (Matsuba et al., J.Biochem.118, S.265-270 (1995)) werden monoklonale Antikörper nicht erwähnt.

Nachdem die Molekülstruktur des TSH-Rezeptors aufgeklärt war, wurden mit dem Ziel der Aufklärung der für die TSH-Bindung und die Antikörperbindung zuständigen Epitope des TSH-Rezeptors von zahlreichen Arbeitsgruppen monoklonale und polyklonale Antikörper gegen vollständige rhTSHR-Polypeptide, gegen den (ohne das Signalpeptid 398 Aminosäuren umfassenden) N-terminalen extrazellulären Teil solcher Rezeptoren und gegen Konjugate kürzerer Rezeptorpeptid-Teilsequenzen hergestellt (vgl. z.B. N.G.Morgenthaler at al., J Clin Endocrinol Metab 81: 3155-3161 (1996); Seetharamaiah GS et al., Endocrinology 134, No. 2, S. 549-554 (1994); Desai RK et al., J. Clin. Endocrinol. Metab. 77:658-663, 1993; Dallas JS et al., Endocrinology 134, No. 3, S. 1437-1445 (1994); Johnstone AP et al., Mol. Cell. Endocrinol. 105 (1994), R1-R9; Seetharamaiah GS et al., Endocrinology 136, No. 7, S. 2817-2824 (1995); Nicholson LB et al., J. Mol. Endocrinol. (1996) 16, S. 159-170; Ropars A et al., Cell. Immunol. 161, S.262-269 (1995); Ohmori M et al., Biochem. Biophys. Res. Commun. 174, No.1 (1991), S.399-403; Endo T. et al., Biochem. Biophys. Res. Commun. 181, No.3 (1991), S.1035-1041; Costagliola S et al., Endocrinology 128, No.3, S.1555-1562, 1991; Marion S et al., Endocrinology 130, No.2, S.967-975 (1992); J. Sanders et al., J. Endocrinol. Invest. 19 (Suppl. No.6); 1996 und weitere, in den genannten Literaturstellen zitierte Veröffentlichungen). Die verschiedenen Antikörper wurden auf ihr Bindungsverhalten gegenüber dem TSH-Rezeptor bzw. rekombinant erzeugten Teilsequenzen davon und insbesondere auch im Hinblick auf ihre Fähigkeit, die Bindung von TSH an verschiedene Formen bzw. Fragmente des TSH-Rezeptors zu stören, geprüft. Da die verschiedenen polyklonalen oder monoklonalen Antikörper durch Immunisierung unterschiedlicher Tiere und/oder unter Verwendung von rekombinantem Material aus unterschiedlichen Expressionssystemen erzeugt worden waren und außerdem bei den Bindungstests häufig rekombinant erzeugte TSH-Rezeptoren unterschiedlichen Ursprungs bzw. Teilpeptide davon verwendet wurden, und da es sich ferner herausstellte, daß für die Bindung zahlreicher Antikörper die Glykolisierung und/oder korrekte Faltung der Rezeptorpeptide entscheidend sein dürfte, ist die Epitopstruktur von nativen TSH-Rezeptoren und das epitopspezifische Bindungsverhalten der in den polyklonalen Autoantikörperpopulationen humaner Seren vorkommenden Autoantikörpern noch nicht umfassend geklärt.

In der Veröffentlichung Dallas JS et al., Endocrinology 134, No. 3, S. 1437-1445 (1994) wird beispielsweise ein mit Hilfe des Baculovirus/Insektenzellen-Expressionssystems hergestellter partieller rekombinanter TSH-Rezeptor, der die Aminosäuren der extrazellulären Domäne des humanen TSH-Rezeptors ohne das N-terminale Signalpeptid aufweist, dazu verwendet, Kaninchen zu immunisieren, und aus den gebildeten Immunglobulinfraktionen werden affinitätschromatographisch unter Verwendung synthetischer Peptide mit jeweils ca. 20 Aminosäuren spezifische Antikörperfraktionen gewonnen. Diese werden dann u.a. auf ihre Eignung untersucht, in einem kommerziellen Rezeptorbindungsassay die Bindung von TSH einen solubilisierten porcinen TSH-Rezeptor zu blockieren. Die Antikörper zeigten keine stimulatorische Aktivität.

In der Veröffentlichung Seetharamaiah GS et al., Endocrinology 136, No. 7, S. 2817-2824 (1995) wird beschrieben, wie der gleiche partielle rekombinante TSH-Rezeptor wie in der vorstehenden Veröffentlichung zur Immunisierung von Mäusen und zur Herstellung von monoklonalen Antikörpern gegen einzelne Epitope des TSH-Rezeptors nach Standardtechniken eingesetzt wurde. Eine ähnliche Vorgehensweise ist beschrieben in Nicholson LB et al., J. Mol. Endocrinol. (1996) 16, S. 159-170.

Gemäß Seetharamaiah GS et al., Endocrinology 134, No. 2, S. 549-554 (1994). wird ein wie vorstehend hergestellter partieller rekombinanter TSH-Rezeptor nachträglich gefaltet und nunmehr auf seine Eignung getestet, radioaktiv markiertes bTSH zu binden. Dazu wird er in flüssiger Phase mit radioaktiv markiertem bTSH umgesetzt. Um den entstanden Komplex möglichst quantitativ aus der Reaktionsmischung abzutrennen, wird dann als Teil eines Fällungssystems ein Antikörper zugesetzt; der durch Immunisierung von Kaninchen mit einem Konjugat eines Teilpeptids, das die Aminosäuren 357 bis 372 der vollständigen TSH-Rezeptorsequenz enthält, erzeugt wurde und von dem festgestellt worden war, daß er die Bindung von bTSH an den ungefalteten partiellen rekombinanten TSH-Rezeptor nicht inhibierte (Desai RK et al., J. Clin. Endocrinol. Metab. 77:658-663, 1993). Der zugesetzte Antikörper bzw. die ihn und gebundenes radioaktiv markiertes bTSH enthaltenden Komplexe werden dann mit Hilfe von Protein A, das unspezifisch an jegliche Antikörper bindet, ausgefällt. Unter den Versuchsbedingungen scheint die Bindung von Protein A an den rezeptorgebundenen Antikörper die gleichzeitige bTSH-Bindung nicht zu beeinträchtigen.

In der älteren, nicht vorveröffentlichten Patentanmeldung WO98/26294 wird ferner erstmals ein praktisch funktionsfähiger kompetitiver Festphasen-Rezeptorassay zur Bestimmung von TSHR-Auto-Ab beschrieben, bei'dem so gearbeitet wird, daß man die. zu bestimmenden TSHR-Auto-Ab und markiertes bTSH bzw. ggf. auch einen markierten monoklonalen Antikörper um Bindungsstellen eines solubilisierten TSHR konkurrieren läßt und die gebildeten TSHR-Komplexe mittels eines immobilisierten monoklonalen Antikörpers an eine Festphase bindet. In der Anmeldung WO98/26294 werden dabei solche monoklonalen Antikörper, die relativ kurze Aminosäure-Sequenzen des TSHR erkennen, in Verbindung mit cruden solubilisierten porcinen oder ggf. auch rekombinanten TSHR-Präparaten verwendet. Die Immobilisierung der TSHR-Komplexe erfolgt im Ausführungsbeispiel mit Hilfe einer Affinitätsgels, an das ein sequentieller monoklonaler Anti-hTSHR-mAb gebunden ist. Das Schwergewicht der Lehre der Anmeldung WO98/26294 liegt auf der Erhöhung der klinischen Wertigkeit der TSHR-Auto-Ab Bestimmung, insbesondere bei Verwendung der herkömmlichen cruden solubilisierten TSHR-Präparationen. Auf den Inhalt der genannten Anmeldung wird, insbesondere was mögliche Assayvariationen angeht, jedoch ergänzend ausdrücklich Bezug genommen.

Ein besonderes Verfahren zur Gewinnung von Anti-hTSHR-mAb ist ferner beschrieben von S.Costagliola und G. Vassart in J.Endocrinol.Invest. 20 (Suppl. to no.5), Abstract 4, (1997). Gemäß diesem Verfahren erfolgt eine Immunisierung von Mäusen zum Zwecke der Antikörperbildung nicht mit einem Antigen peptidischer Natur, sondern durch intramuskulare Injektion eines für den hTSHR kodierenden DNA-Plasmidkonstrukts. Es werden auf diese Weise neue monoclonale Antikörper mit hoher Affinität zum nativen hTSHR erhalten. Diese Technik hat sich als sehr wertvoll erwiesen und wird auch im Rahmen der vorliegenden Anmeldung insbesondere zur Herstellung von Anti-hTSHR-mAb genutzt, die konformationelle Epitope erkennen.

Ergänzend sei angemerkt, daß die mit' Antikörpern gegen rekombinante" TSH-Rezeptoren oder deren Teile gewonnenen Erkenntnisse zu dem Vorschlag führten, zur Bestimmung von TSH-Rezeptor-Autoantikörpern ein drittes, an sich bekanntes Verfahrensprinzip in Form eines Immunpräzipitationsassays zu nutzen, bei dem als Reagens zur Fällung eine Präparation eines extrazellulären Teils eines rekombinanten, durch Einbau von ³⁵S-Methionin markierten TSH-Rezeptors verwendet wird. Bei einem solchen Assay besteht weder eine Selektivität für TSI noch für TBII. (N.G. Morgenthaler at al., J Clin Endocrinol Metab 81: 700-706 (1996)). Die Herstellung des ³⁵S-markierten Rezeptors durch in vitro-Translation ist jedoch außerordentlich aufwendig und teuer, und es gibt keine Meßgeräte, die für eine klinische Routinemessung der von ³⁵S emittierten Strahlung geeignet sind. Das Verfahren ist daher als Meßverfahren für die klinische Routinediagnostik nicht geeignet.

Es ist Aufgabe der vorliegenden Erfindung, einen Rezeptorbindungsassay zur Bestimmung von TSH-Rezeptor-Autoantikörpern zu schaffen, der die beschriebenen Nachteile derartiger kompetitiver Rezeptorbindungsassays des Standes der Technik nicht aufweist und von hoher klinischer Wertigkeit ist.

Insbesondere ist es Aufgabe der vorliegenden Erfindung, einen verbesserten Rezeptorbindungsassay zur Bestimmung von TSH-Rezeptor-Autoantikörpern zu schaffen, bei dem die aus den Reaktionspartnern des Bestimmungsverfahrens gebildeten TSH-Rezeptor-Komplexe direkt in festphasengebundener Form erhalten werden, so daß auch eine automatisierte Durchführung derartiger Rezeptorbindungsassays möglich wird.

Insbesondere ist es ferner Aufgabe der vorliegenden Erfindung, verbesserte Rezeptorbindungsassays zum Nachweis von TSH-Rezeptor-Autoantikörpern so zu gestalten, daß bestimmte Störungen der Messung durch anomale Serumbestandteile effektiv ausgeschaltet werden und eine optimale Bindung der Reaktionspartner des Bestimmungsverfahrens gewährleistet ist.

Es ist ferner Aufgabe der vorliegenden Erfindung, die zur Durchführung derartiger verbesserter Rezeptorbindungsassays in der klinischen Routinediagnostik erforderlichen Reagenziensätze (Kits) zu schaffen.

Die genannten Aufgaben werden bei einem kompetitiven Rezeptorbindungsassay gemäß Oberbegriff von Anspruch 1 wenigstens teilweise durch Radiorezeptprassays gelöst, die die im Kennzeichen des Anspruchs 1 wiedergegebenen Merkmale aufweisen.

Vorteilhafte und Zweckmäßige Weiterbildungen des erfindungsgemäßen Verfahrens sind in den Ansprüchen 2-12 gekennzeichnet.

Im Rahmen der zur Lösung der o.g. Aufgabe führenden Untersuchungen wurden ferner verschiedene Erkenntnisse gewonnen, die die Bindung von TSH, insbesondere bTSH, an rhTSHR betreffen und in die nachfolgend im experimentellen Teil beschriebenen Tests eingegangen sind.

Derartige vorteilhafte Ausgestaltungen der erfindungsgemäßen verbesserten Rezeptorbindungsassays sind auch Unteransprüchen zu entnehmen, insbesondere in Verbindung mit den detaillierteren Erläuterungen in der nachfolgenden Beschreibung.

Die Aufgabe der Schaffung eines Reagenziensatzes zur Verwirklichung der vorliegenden Erfindung wird durch einen bevorzugten Reagenziensatz gelöst, der wenigstens jeweils einen der Bestandteile (i) und (ii) gemäß Anspruch 13 enthält.

In der Einleitung und im nachfolgenden Teil dieser Anmeldung werden die verwendeten Reagenzien bzw. Analyten/Biomoleküle in der Regel mit verschiedenen Abkürzungen gekennzeichnet, die stets in den folgenden Bedeutungen zu verstehen sind, es sei denn, es ergibt sich aus dem konkreten Zusammenhang für den Fachmann ausnahmsweise etwas anderes. Die Verwendung der speziellen Angaben erfolgt dabei aus Gründen der exakten Beschreibung der durchgeführten Versuche und Messungen, bedeutet jedoch nicht, daß die beschriebenen Ergebnisse und Schlüßfolgerungen nur für den beschriebenen Spezialfall gelten. Zahlreiche der vermittelten Informationen sind vielmehr für den Fachmann in ihrer allgemeineren Bedeutung klar erkennbar.

Erläuterungen zu den verwendeten Abkürzungen:
- TSH =: Thyroidstimulierendes Hormon (Thyreotropin). Wird die Abkürzung TSH ohne weitere Zusätze verwendet, handelt es sich nicht um ein bestimmtes Produkt, sondern die Bindung bzw. Funktion des Hormons wird in allgemeiner Form diskutiert.
- bTSH =: Bovines (d.h. aus Rindern gewonnenes) TSH. Präparat, das in Assays zur Bestimmung von Autoantikörpern gegen den TSH-Rezeptor als Tracer eingesetzt wird (insbesondere radioiodiert oder, wie hierin beschrieben, mit einem Chemilumineszenzlabel, insbesondere einem Acridiniumesterlabel, markiert; es liegt jedoch im Rahmen der vorliegenden Erfindung, irgendein bekanntes anderes Label zu verwenden).
- ¹²⁵I-bTSH =: Radioiodiertes, als Kompetitor eingesetztes bTSH. Im Zusammenhang mit der Beschreibung von Assays der Firma B.R.A.H.M.S Diagnostica GmbH steht ¹²⁵I-bTSH insbesondere für ein Produkt, wie es gemäß DE 42 37 430 C1 bzw. EP 0 668 775 B1 erhalten wird und Bestandteil des TRAK-Assay® der Fa. B.R.A.H.M.S Diagnostica GmbH ist.
- hTSH =: Humanes TSH. Kommt im Serum/Plasma Gesunder nur in sehr geringen Konzentrationen 0,2-4 mU/l vor. In Seren von hypothyreoten Patienten kann die hTSH-Konzentration jedoch signifikant erhöht sein. Die durch erhöhte hTSH-Spiegel (von mehr als 20 mU/l) hervorgerufenen Störungen von Autoantikörper-Messungen werden in der Beschreibung diskutiert und durch spezielle Maßnahmen neutralisiert. Der gegenwärtige Kenntnisstand zur Struktur von hTSH findet sich zusammengefaßt in Grossmann et al., Endocrine Reviews, Vol.18, 1997, S. 476-501.
- TSHR =: Der TSH-Rezeptor, ein in der Schilddrüsenmembran verankerter Glykoproteinrezeptor. Wird die Abkürzung TSHR ohne weitere Zusätze verwendet, handelt es sich nicht um ein bestimmtes Produkt, sondern die Funktion des Rezeptors bzw. seine Bindungsbeteiligung wird in allgemeiner Form diskutiert.
- porc.TSHR =: Aus Schilddrüsen von Schweinen (porcines) extraktiv gewonnenes solubilisiertes crudes Rezeptorpräparat. Wird in den Radiorezeptor-Präzipitationsassays des Standes der Technik zur Bestimmung von Autoantikörpern gegen den TSHR (herkömmlicher TRAK-Assay® der Fa. B.R.A.H.M.S Diagnostica GmbH) als spezifischer Binder eingesetzt.
- rhTSHR =: Gentechnisch erzeugtes (rekombinantes) Polypeptid, das die Aminosäuresequenz eines natürlich vorkommenden humanen TSHR wenigstens in einem solchen Ausmaße aufweist, daß es als "funktionaler humaner TSH-Rezeptor" bezeichnet werden kann, was bedeutet, daß es sich bezüglich der Bindung von Autoantikörpern gegen den TSHR bzw. von hTSH in signifikantem Ausmaß wie der natürlich vorkommende humane TSHR verhält. Wird die Abkürzung rhTSHR ohne weitere Zusätze verwendet, handelt es sich nicht um ein bestimmtes Produkt, d.h. rhTSHR kann für irgendein rekombinantes vollständiges, mehr oder weniger glykosyliertes Polypeptid, eine Teilsequenz einer ausreichenden Länge oder ein gentechnologisch erzeugtes Fusionsprodukt davon stehen (wie es z.B. in der Internationalen Patentanmeldung PCT/EP97/06121 beschrieben wird). Ohne weitere Erläuterungen liegt ein einfach als "rhTSHR" bezeichnetes Produkt als crude detergenssolubilisierte Membranpräparation vor, d.h. in der Form, wie sie durch konventionelle Solubilisierung von Membranen der zur Expression des rekombinanten Polypeptids verwendeten Zellen unter Verwendung von Detergenzien erhalten wird.
- rhTSHR(imm) =: Selektiv an eine Festphase gebundenes (immobilisiertes) rhTSHR-Präparat. Die Bindung kann - wie in der Beschreibung nachfolgend genauer beschrieben - über einen geeigneten Antikörper erfolgen, kann aber im Falle von Fusionsprodukten auch über einen besonderen Peptidrest, z.B. einen Biotinrest, erfolgen. Die Festphase kann die Wand eines Teströhrchens (Coated-Tube- oder CT-Technik) sein, kann aber auch eine geeignete suspendierte Festphase sein.
- rhTSHR(imm)* =: Über einen ausgewählten Anti-hTSHR-mAb an eine Festphase (s.o.) gebundener und in gebundener Form durch Waschen von Fremdbestandteilen gereinigter immobilisierter rhTSHR. Wird auch als "affinitätsgereinigter rhTSHR" bezeichnet.
- Ab: = Antikörper
- TSHR-Auto-Ab: = In biologischen Proben, insbesondere humanem Serum oder Plasma, nachweisbare Autoantikörper gegen den TSH-Rezeptor. Der Nachweis stimulierender derartiger TSHR-Auto-Ab (in der Literatur auch abgekürzt TSI = thyroid stimulating immunglobulins) ist insbesondere für die Diagnose des Morbus Basedow (englisch: Graves' disease) von Bedeutung. Ein kommerzieller Assay (Radiorezeptorassay) zur Bestimmung von TSHR-Auto-Ab ist der TRAK-Assay® der Fa. B.R.A.H.M.S Diagnpstica GmbH.
- Anti-hTSHR-mAb =: Monoklonaler Antikörper, der an rhTSHR bindet. Ohne weitere Erläuterungen kann er, was sein Bindungsverhalten angeht, sequentieller Natur sein, wie z.B. in der älteren Anmeldung DE 196 51 093.7 beschrieben wird, er kann jedoch auch konformativer Natur sein. Die Selektion bestimmter derartiger Anti-hTSHR-mAb für die Verwirklichung der erfindungsgemäßen Assays wird in der Anmeldung näher beschrieben. Werden in den Versuchen spezielle Anti-hTSHR-mAb verwendet, werden sie durch eine in der Anmeldung erläuterte Kennummer gekennzeichnet. Wird eine leicht abgewandelte, weniger spezifische Abkürzung (z.B. einfach Anti-TSHR-Ab) verwendet, soll eine an dieser Stelle unnötige Beschränkung auf monoklonale Ab gegen den humanen TSHR vermieden werden.
- Anti-bTSH-Ab =: In humanen Seren bzw. Plasma vorkommende Antikörper ungeklärten Ursprungs, die mit bTSH unter Bildung von Immunkomplexen reagieren und damit die Bindung von bTSH an die Assaykomponenten bzw. das erhaltene, Meßergebnis beeinflussen (vgl. Y.Ochi et al., Acta Endocrinologica (Copenh) 1989, 120: 773-777; S.Sakata et al., J.Endocrinol. Invest. 14: 123-130, 1991; T. Inui, Thyroid, Vol.6: 295-299, 1996).

Wie im einleitenden Teil erläutert wurde, enthalten die bekannten kompetitiven Rezeptorbindungsassays, die alle als Radiorezeptorassays ausgestaltet sind, neben den benötigten Standards und Pufferlösungen folgende grundlegenden Assaybestandteile:
(i) Eine TSH-Rezeptor-Präparation als spezifischen Binder, in den herkömmlichen Assays insbesondere einen aus Schweine-Schilddrüsenmembranen gewonnenen solubilisierten nativen porcinen TSH-Rezeptor (porc.TSHR), (ii) ¹²⁵I-bTSH, das mit TSH-Rezeptor-Autoantikörpern aus einer Serumprobe oder Plasmaprobe (TBII) um gemeinsame Bindungsstellen an. dem verwendeten TSH-Rezeptor konkurriert und (iii) ein Mittel zur Abtrennung des gebildeten TSHR-Komplexes von der flüssigen Reaktionslösung, das im Falle des herkömmlichen Assay das Fällungsmittel Polyethylenglykol (PEG) ist.

Es ist ein die vorliegende Erfindung gegenüber dem bekannten Stand der Technik auszeichnendes Merkmal, daß experimentell gezeigt wurde, daß es möglich ist, anstelle einer solubilisierten TSHR-Präparation einen funktionalen rhTSHR (d.h. einen humanen rekombinanten TSH-Rezeptor) in affinitätsgereinigter Form zu verwenden, der selektiv an einer Festphase immobilisiert ist, ohne daß die Bindungsfähigkeit des affinitätsgereinigten rhTSHR (rhTSHR(imm)*) gegen die zu bestimmenden TSHR-Auto-Ab und markiertes bTSH nennenswert beeinträchtigt ist. Mit dieser Erkenntnis wurde die Voraussetzung geschaffen, einen in mehrfacher Hinsicht noch weiter verbesserten Rezeptorbindungsassay zur Bestimmung von TSHR-Auto-Ab zu entwickeln, bei dem die Bestimmung der Menge des gebundenen markierten bTSH direkt in festphasengebundener Form erfolgen kann. Bei der genaueren Untersuchung der Bindung von bTSH an die neuartige TSHR-Präparation wurden zusätzliche wertvolle Erkenntnisse gewonnen, die in die Erfindung bzw. ihre bevorzugten Ausführungsformen Eingang gefunden haben. Nähere Einzelheiten sind dem experimentellen Teil zu entnehmen. Die wichtigsten der genannten weiteren Erkenntnisse sind:
1. Der Störeinfluß von pathologisch hohen hTSH-Konzentrationen in einzelnen Patientenproben läßt sich durch Zusatz von bestimmten kommerziell erhältlichen Antikörpern (Anti-hTSH-Ab) zur probenhaltigen Meßlösung neutralisieren, die selektiv hTSH binden und nicht mit dem als Kompetitor eingesetzen bTSH kreuzreagieren.
2. Wenn man die Bestimmung als "Zweischritt-Bestimmung" durchführt, indem man markiertes bTSH in einem nachgeschalteten Schritt mit einem vorher gebildeten und von der ursprünglichen Meßlösung abgetrennten Komplex aus TSHR-Auto-Ab und dem affinitätsgereinigten rhTSHR(imm)* reagieren läßt, kann sich eine mögliche Anwesenheit von Anti-bTSH-Ab in der Patientenprobe nicht mehr störend auswirken.
3. Die oben unter 2. aufgeführte neue Erkenntnis ermöglicht es, die Umsetzung mit bTSH im zweiten Assayschritt mit einem exakt standardisierbaren, von Serum freien bTSH-Reagens durchzuführen.
4. Für die Bindung von bTSH an rhTSHR-Präparationen ist die Anwesenheit von Drittsubstanzen vorteilhaft, z.B. von bestimmten Serumbestandteilen, die als Bestandteile einer Serumfraktion, die nur Substanzen mit Molekulargewichten von <10.000 d enthält, charakterisiert werden konnten. Einige der niedermolekularen Substanzen, die die Bindung verbessern, insbesondere gelöste anorganische Ionen, konnten genauer identifiziert werden. Es konnte gezeigt werden, daß anorganische Ionen jedoch bei Komplexierung durch EDTA, d.h. in festen Komplexen gebunden, die genannte Wirkung nicht aufweisen.

Dadurch, daß das erfindungsgemäße Verfahren eine Bindung der TSHR-Auto-Ab und des markierten Kompetitors bTSH aus der Meßlösung direkt an einen festphasengebundenen, affinitätsgereinigten rhTSHR(imm)* gestattet, wird nicht nur die Messung des erhaltenen Signals gegenüber einem Präzipitationsassay im gewünschten Sinne praktisch vereinfacht, sondern das Assaydesign kann grundsätzlich verändert werden, indem man von einem Einschritt-Assay (eine einzige, durch aufeinanderfolgende Pipettierungen ohne zwischenzeitliche Fest-Flüssig-Trennung erhaltene Meßlösung) zu einem Zweischritt-Assay übergeht, bei dem man die Umsetzung von rhTSHR(imm)* mit der Probe und die Umsetzung mit dem markierten Kompetitor bTSH in zwei aufeinanderfolgenden, durch eine Fest-Füssig-Trennung voneinander abgesetzten Schritten durchführt.

Als Festphasenträger für den affinitätsgereinigten rhTSHR(imm)* können Kunststoffoberflächen, insbesondere die Wände von Kunststoff-Teströhrchen für die CT-Technik, Mikropartikel, Magnetpartikel, Filter, Polymergelmaterialien und andere bekannte Festphasenträger eingesetzt werden. Durch das erfindungsgemäße Verfahren wird die TSHR-Auto-Ab-Bestimmung auch automatisierbar. So kann das Assaydesign so angepaßt werden, däß es an bekannten automatisierten Systemen durchführbar ist (vgl. z.B. Elecsys-System der Firma Boehringer Mannheim oder ACS 180-System von Chiron). Bei derartigen automatisierten Systemen werden im Rahmen einer automatischen Abarbeitung die Proben in rezeptorbeschichtete Teströhrchen pipettiert, inkubiert, dann wird die flüssige Reaktionsmischung abgesaugt oder dekantiert, und es wird eine zweite Lösung mit dem markierten bTSH, z.B. ¹²⁵I-bTSH, zugesetzt. Nach der vorgeschriebenen Inkubation erfolgt dann die übliche abschließende Fest-Flüssigtrennung, wonach das Signal, gegebenenfalls nach Signalauslösung durch Zugabe geeigneter Reagenzien, gemessen werden kann. Die beispielhaft für einen Zweischritt-Assay angegebene Pipettierschrittfolge ist dabei variabel und z.B. durch die Pipettierfolge eines Einschritt-Assays (2.1.1), ggf. auch ohne vorherige Immobilisierung des rTHSR, ersetzbare.

Nachfolgend wird die vorliegende Erfindung in ihren verschiedenen Aspekten anhand von konkreten Ausführungsbeispielen und Versuchsergebnissen unter Bezugnahme auf acht Figuren noch näher erläutert. Auf die allgemeinen Erläuterungen im Zusammenhang mit den beschriebenen Versuchen wird ausdrücklich als Teil der Offenbarung der vorliegenden Erfindung verwiesen.

Dabei zeigen die Figuren in Form verschiedener Diagramme:
- Figur 1:: Die Abhängigkeit der Bindung von ¹²⁵I-bTSH an einen affinitätsgereinigten rhTSHR(imm)*, der in einem vorgeschalteten Umsetzungsschritt mit Serum mit steigenden Anteilen hTSH behandelt worden war (Kurvenzug -■-), wobei ein Teil der Seren (Kurvenzug -▲-) zusätzlich einen Überschuß eines selektiven Anti-hTSH-Ab enthielt;
- Figur 2:: Die Standardkurve für die Messsung von TSHR-Auto-Ab unter Verwendung von ¹²⁵I-bTSH und von einem affinitätsgereinigten rhTSHR(imm)* in einem Zweischritt-Verfahren.
- Figur 3:: Die Standardkurve für die Messsung von TSHR-Auto-Ab unter Verwendung von einem mit einem Akridiniumester chemilumineszenz-markierten bTSH und von einem affinitätsgereinigten rhTSHR(imm)* in einem Zweischritt-Verfahren.
- Figur 4:: Die Ergebnisse der Messung von Patientenseren (100 Kontrollseren; 70 Seren von Morbus Basedow-Patienten) nach dem Zweischritt-Verfahren unter Verwendung von ¹²⁵I-bTSH und affinitätsgereinigtem rhTSHR(imm)*.

Die nachfolgenden Beschreibungen von Herstellungs- und Meßexperimenten gliedern sich wie folgt:
1. Herstellung/Auswahl der verwendeten Assaykomponenten;
2. Inkubationsprotokolle für 2 Meßverfahren unter Verwendung von markiertem bTSH und affinitätsgereinigtem rhTSHR(imm)* nach einer Einschritt- und Zweischritt-Technik; und
3. Ergebnisse der Messung von TSHR-Auto-Ab in Standardproben bzw. Patientenseren unter Verwendung von erfindungsgemäßen Teströhrchen, die mit affinitätsgereinigten rhTSHR(imm)* beschichtet sind, nach dem Prinzip der Kompetition mit markiertem bTSH.

### 1. Materialien

### 1.1 Herstellung einer Präparation eines kruden solubilisierten rhTSHR

Um den rhTSHR in hoher Ausbeute in einer Säugetierzellinie zu exprimieren, wurde eine humane chronische Leukämiezellinie analog zu gängigen Arbeitsweisen mit einem bicistronischen Vektor, der die cDNA für den vollständigen humanen TSH-Rezeptor enthielt, stabil transfiziert und in Suspensionskultur gezüchtet. Orientierende Versuche mit anderen Systemen zeigten, daß das spezielle Expressionssystem nicht kritisch ist und mit im wesentlichen gleichem Erfolg auch mit dem in EP-A-0 719 858 beschriebenen, mit Myelom-Zellinien arbeitenden Expressionssystem oder mit dem in WO98/20343 beschriebenen Vacciniavirus/HeLa-Zellen-Expressionssystem gearbeitet werden kann. Auch mit den in EP-B1-0 433 509 beschriebenen Warmblüterzellen konnte der rhTSHR in einer Qualität erhalten werden, die eine Durchführung der Lehre dieser Anmeldung ermöglichte. Aufgrund der bisherigen Erkenntnisse ist es jedoch wichtig, daß die Expression des humanen TSH-Rezeptors in Säugetierzellen erfolgt, wobei vorzugsweise solche zu verwenden sind, die in Suspension gezüchtet werden können und hohe rhTSHR-Ausbeuten liefern.

Zellen, die den exprimierten rhTSHR in ihrer Zellmembran enthielten, wurden zur Gewinnung einer solubilisierten rhTSHR-Präparation aufgeschlossen, wie genauer beschrieben wird in S. Costagliola et al., J. Clin. Endocrinol. Metab. 75: 1540-1544 (1992), wobei alle Arbeitsschritte bei 4°C durchgeführt wurden. Und zwar wurden jeweils 10⁹ Zellen in 50mM HEPES (4-(2-Hydroxyethyl)-1-piperazin-ethansulfonsäure); pH 7,5 aufgeschlossen. Der Aufschluß erfolgte unter Zuhilfenahme eines Potter-Homogenisators (Fa. B Braun Melsungen AG) durch zehnfaches Auf- und Abbewegen des Kolbens bei einer Drehzahl von 900 U/min. Das erhaltene Material wurde bei 100.000 g 30 min zentrifugiert. Das erhaltene Sediment wurde in 20 ml Puffer (10 mM HEPES; 2% Triton-X-100; pH 7,5) unter Wiederholung der beschriebenen Arbeitsweise rehomogenisiert und wiederum 30 min bei 100.000 g zentrifugiert. Der erhaltene Überstand, der die krude solubilisierte rhTSHR-Präparation enthielt, wurde portioniert und bis zur weiteren Verwendung bei -80°C aufbewahrt.

### 1.2 Herstellung von markiertem bTSH

### 1.2.1 ¹²⁵I-bTSH

¹²⁵I-bTSH wurde wie in den Patenten DE 42 37 430 C1 bzw. EP 0 668 775 B1 beschrieben, hergestellt.

### 1.2.2 Mit Akridiniumester chemilumineszenz-markiertes bTSH

Mit Akridiniumester chemilumineszenz-markiertes bTSH wurde wie folgt hergestellt: 100 µg bTSH (50-60 IU/mg Protein; in 20 mM Natriumphosphat; pH 7,0) wurden mit 10 µl Akridiniumester (Fa. Behringwerke AG, Marburg; vgl. EP 0 257 541 B1; 1 mg/ml in Acetonitril) für 15 min bei Raumtemperatur umgesetzt und anschließend mittels HPLC an einer Waters-Protein Pak SW 125-Säule präpariert (Laufmittel: 0,1 M Ammoniumacetat; pH 5,5; Flußrate: 0,6 ml/min). Alle Fraktionen des Säulenausflusses mit einer UV-Absorption bei 280 nm und 368 nm wurden gesammelt. Die gepoolte Proteinfraktion wurde bis zur weiteren Verwendung in Rezeptorbindungsassays bei -80°C gelagert.

### 1.3 Standards

Standards für die Bestimmung von TSHR-Auto-Ab wurden durch Mischen von TSHR-Auto-Ab-positiven Seren mit bekanntem Antikörpergehalt und Autoantikörper-freien Humanseren hergestellt, jeweils auf 0,05% Natriumazid eingestellt und bis zur weiteren Verwendung bei 4°C gelagert. Die Kalibrierung der Standards erfolgte am TRAK-Assay® der Firma B.R.A.H.M.S Diagnostica GmbH.

### 1.4 Anti-hTSHR-mAb-Gewinnung und Selektion

Es wurde ein Kollektiv monoklonaler Maus-Antikörper gegen den TSH-Rezeptor unter Anwendung verschiedener Immunisierungsmethoden wie in der Literatur beschrieben, hergestellt (J.S. Dallas et al., Endocrinology 134, No. 3, S. 1437-1445 (1994) - Immunisierung mit nach dem Baculovirus-Verfahren rekombinant hergestellter extrazellulärer Rezeptordomäne; L.B. Nicholson et al., J. Mol. Endocrinol. (1996) 16, S. 159-190 - Immunisierung mit der in Prokaryonten-Zellen oder nach dem Baculovirus-Verfahren erzeugten extrazellulären Rezeptordomäne; Johnstone A.P. et al., Mol. Cell. Endocrinol. 105 (1994), R1-R9; - Immunisierung mit verschiedenen rekombinanten TSHR-Polypeptiden; Immunisierung mit synthetischen Rezeptorpeptid-Konjugaten; J. Sanders et al., J. Endocrinol. Invest. 19 (Suppl. to No. 6): 1996, Abstract 33 - Immunisieren mit in E.Coli erzeugten, TSHR-Fragmente enthaltenden Fusionsproteinen; sowie insbesondere S. Costagliola und G. Vassart, J. Endocrinol. Invest. 20 (Suppl. to No. 5): 1997, Abstract No. 4 - Immunisierung von Mäusen mit einem DNA-Konstrukt).

Die Selektion sowie Gewinnung einzelner Klone erfolgte wie in den jeweiligen zitierten Literaturstellen beschrieben bzw. nach allgemein bekannten Verfahren zur Herstellung monoklonaler Antikörper. Die Reinigung der verschiedenen erhaltenen monoklonalen Antikörper erfolgte mittels Protein A-Affinitätschromatographie. Elf der erhaltenenen Anti-hTSHR-mAb wurden auf ihre Fähigkeit hin getestet, einen vorher erzeugten Komplex aus rhTSHR und ¹²⁵I-bTSH zu binden.

Dazu wurde in einem ersten Schritt ¹²⁵I-bTSH (36.000 cpm Totalaktivität) mit dem solubilisierten rhTSHR (ca. 1 ng) in einem Gesamtvolumen von 100 µl inkubiert, das 50 mM HEPES; pH 7,5; 10.000 IU/ml Heparin; 10 mM CaCl₂; 0,2% Triton X-100 enthielt (vgl. die nachfolgenden Versuchsergebnisse). Zur Kontrolle der unspezifischen Bindung wurde in separaten Ansätzen je 0,1 IU/ml unmarkiertes bTSH zugesetzt. Nach einer einstündigen Inkubation bei Raumtemperatur erfolgte die Bestimmung der Bindung bTSH/rhTSHR nach drei unterschiedlichen Methoden:

### 1.4.1 Isolierung von vorher gebildeten bTSH/rhTSHR-Komplexen mittels PEG-Präzipitation (Gewinnung eines Bezugswerts)

Der obigen Reaktionslösung wurden 2 ml PEG-Fällungsreagenz aus dem TRAK-Assay® der Firma B.R.A.H.M.S Diagnostica GmbH zugesetzt. Anschließend wurde zentrifugiert (10 min bei 2.000 g), der Überstand wurde dekantiert, und es wurde die im Sediment verbliebene Radioaktivität gemessen. Es wurde eine quantitative Fällung des gebildeten bTSH/rhTSHR-Komplexes festgestellt.

### 1.4.2 Immunpräzipitation von vorher gebildeten bTSH/rhTSHR-Komplexen mit den zu prüfenden Anti-hTSHR-mAb

Zu der obigen Reaktionslösung wurden jeweils 10 µg (in 50 µl des zur Erzeugung des bTSH/rhTSHR-Komplexes verwendeten Puffers) des jeweiligen zu untersuchenden Anti-hTSHR-mAb zugesetzt, und nach einer einstündigen Inkubation wurden als Fällungsreagenz 100 µl Protein A (aus HENNING-Test® anti-TPO der Firma B.R.A.H.M.S Diagnostica GmbH) zugegeben, 15 min inkubiert und schließlich 2 ml phosphatgepufferte Salzlösung (PBS) zugegeben, wonach zentrifugiert wurde (15 min bei 2.000 g). Durch die Zugabe von Protein A werden alle in der Reaktionsmischung enthaltenen Antikörper ausgefällt. Der bTSH/rhTSHR-Komplex wird nur insoweit ausgefällt, als er an den zu untersuchenden Antikörper gebunden hat. Demzufolge repräsentiert die im Zentrifugationssegment zu findende Radioaktivität die Menge an bTSH/rhTSHR-Komplex, die von dem jeweiligen Anti-hTSHR-mAb gebunden wurde.

### 1.4.3 Bindung von vorher gebildeten bTSH/rhTSHR-Komplexen mit Hilfe immobilisierter Anti-hTSHR-mAb (CT-Technik)

Zur Immobilisierung der jeweiligen Anti-hTSHR-mAbs wurden mit Ziegen-anti-Maus-Antikörper beschichtete Polystyrolröhrchen (Kit-Bestandteil des DYNOtest® TBG der Firma B.R.A.H.M.S Diagnostica GmbH) mit je 100 ng der zu untersuchenden Anti-hTSHR-mAb mit den Kennummern 1 bis 11 in 200µl PBS 2 h bei Raumtemperatur inkubiert. Anschliebend wurden 2 ml PBS zugegeben, und der flüssige Röhrcheninhalt wurde dekantiert. Die erhaltenen beschichteten Teströhrchen wurden dann direkt mit der oben beschriebenen Reaktionslösung inkubiert, die den bTSH/rhTSHR-Komplex enthielt (100 µl), und zwar 1 h unter Schütteln bei 300 U/min. Danach wurden die Teströhrchen zweimal mit je 2ml Waschpuffer (10 mM HEPES; 0,1 Triton X-100; pH 7,5) gewaschen, und die auf der Oberfläche der Teströhrchen fixierte Radioaktivität wurde gemessen. Als vollständige Bindung des bTSH/rhTSHR-Komplexes wird der bei der PEG-Fällung erhaltene Radioaktivitätswert eingesetzt, abzüglich der getrennt ermittelten unspezifischen Bindung: (B-UB) = 10.490 - 433 cpm = 10.057 cpm. Dieser Wert entspricht einem B/T-Wert (gebundene Radioaktivität zu eingesetzter Gesamtradioaktivität) von 28 %.

Die bei der Prüfung der Anti-hTSHR-mAb 1-11 erhaltenen Bindungswerte nach den Verfahren 1.4.3 und 1.4.4 werden in der nachfolgenden Tabelle Nr. 1 angegeben, wobei die Radioaktivität des Sediments der PEG-Fällung gemäß 1.4.1 als Bezugswert mit 100% angesetzt wurde. Bei allen gezeigten Daten sind die unspezifischen Bindungen, d.h. die Radioaktivität in Anwesenheit eines Überschusses an unmarkiertem bTSH, abgezogen.

**Tabelle 1**

| Antikörper-Nummer | Immunpräzipitation mit 10 µg Ab % Bindung * | CT-Assay mit 100 ng Ab % Bindung * | Reaktivität im Westernblot ** | Epitop *** |
|---|---|---|---|---|
| 1 | 40,9 | 4 | + | 22-35 |
| 2 | 11 | < 2 | + | 32-54 |
| 3 | 12,8 | < 2 | + | 287-301 |
| 4 | 10,8 | < 2 | + | 334-343 |
| 5 | 28 | 5,1 | + | 354-359 |
| 6 | 13,4 | 3,5 | + | 352-361 |
| 7 | 41,7 | 8 | + | 361-381 |
| 8 | < 2 | < 2 | + | 402-415 |
| 9 | 99 | 47,6 | - | 3D |
| 10 | 103 | 16,2 | - | 3D |
| 11 | 104 | 20,6 | - | 3D |

| | | | | |
|---|---|---|---|---|
| Erläuterungen zu Tabelle 1: * Die Werte sind jeweils angegeben als prozentuale Fällung des maximal fällbaren bTSH/rhTSHR-Komplexes (100% = Präzipitation bei PEG-Fällung); | | | | |
| ** bestimmt wie beschrieben von Johnstone, A. P. et al., Mol. Cell. Endocrinol. 105 (1994), R1-R9; | | | | |
| *** vgl. die nachfolgenden Versuche zur Kartierung der Bindungsstellen von Anti-hTSHR-mAb (Maus) | | | | |

### 1.4.4. Kartierung der Bindungsstellen von Anti-hTSHR-mAb (Maus)

Das Prinzip der Kartierung beruht darauf, daß festgestellt wird, an welche synthetischen Teilpeptidsequenzen des hTSHR der jeweilige zu untersuchende Antikörper bindet.

### 1.4.4.1 Herstellung eines Kollektivs von Peptiden, die Teilsequenzen des hTSHR entsprechen.

Es wurden jeweils 13 Aminosäuren umfassende Teilsequenzen der hTSHR-Sequenz in Form synthetischer Peptide hergestellt. Die Teilsequenzen wurden dabei so gewählt,daß die gesamte hTSHR-Sequenz abgebildet wird und je zwei aufeinanderfolgende Sequenzen um neun Aminosäuren überlappen (z.B. Peptid 1: Aminosäuren 1 bis 13; Peptid 2: Aminosäuren 4 bis 16; Peptid 3: Aminosäuren 7 bis 19 usw.). Die synthetischen Peptide wurden dann nebeneinander in Form von ca. 2 x 2 mm großen "Spots" auf Zellulosepapier synthetisiert. Dieses Verfahren der Peptidherstellung ist ein etabliertes Verfahren und wird kommerziell u.a. von der Firma JERINI Biotools GmbH, Berlin, angeboten. Zur Erläuterung wird verwiesen auf die folgenden Literaturstellen: R. Frank, Peptides 1990, 151-152; A. Furga et al., Int. J. Peptide Protein Res. 37, 1991, 487-493; R. Frank, Tetrahedron Vol. 48, Nr. 42, 9217-9232, 1992. Das Papier mit den Peptidspots wurde 1 h bei Raumtemperatur in 20 ml sogenannter "Blockierungslösung" inkubiert (Blockierungslösung: 50 mM Tris; pH 8,0; 50 mM NaCl; 0,05% Tween 20; 5% Saccharose; 1 x Blocking Reagent der Firma Cambridge Research Biochemicals, Nr. SU-07-250).

### 1.4.4.2 Charakterisierung der Anti-hTSHR-mAb 1 bis 11

Dem gemäß 1.4.4.1 präparierten Zellulosepapier wurden dann 20 µl einer Lösung zugesetzt, die einen der zu untersuchenden Anti-hTSHR-mAb 1-11 in einer Konzentration von 10 µg/ml enthielt. Anschließend wurde 3h bei Raumtemperatur unter Schwenken inkubiert, danach dekantiert. Das Papier wurde 6 x mit 5 ml "Waschpuffer" gewaschen (Waschpuffer: 50 mM Tris; pH 8,0; 50 mM NaCl; 0,05% Tween). Dann wurden 20 ml der obigen Blockierungslösung zugegeben, die 5µl Ziege-anti-Maus-IgGalkalische Phosphatase-Konjugat der Firma BIORAD, Nr. 172-1015 in frisch verdünntem Zustand enthielt. Es wurde wiederum 2 h bei Raumtemperatur unter Schwenken inkubiert. Dann wurde wiederum dekantiert, und das Papier wurde 6 x mit 5 ml des obigen Waschpuffers gewaschen.

Durch Zugabe einer Substratlösung, die mit der Antikörpergebundenen alkalischen Phosphatase reagiert, wurden schließlich diejenigen Peptide identifiziert, an die der jeweilige untersuchte Anti-hTSHR-mAb gebunden hatte. Die zur Sichtbarmachung verwendete Substratlösung wurde wie folgt erhalten: 6 mg Nitro Blue Tetrazolium (SIGMA Nr. N-6876) wurden in 6 ml Dimethylformamid gelöst. 2,5 mg 5-Brom-4-chlor-3-indolylphosphat (SIGMA-Nr. B-8503) wurden in 0,5 ml Dimethylformamid gelöst. 4 ml der beschriebenen Nitro Blue Tetrazolium-Lösung, 400 µl der 5-Brom-4-chlor-3-indolylphosphatlösung sowie 160 µl einer 1 M MgCl₂-Lösung wurden in 36 ml Substratlösung aus dem ELItest® Anti-TG der Firma B.R.A.H.M.S Diagnostica GmbH, Nr. 944638, gelöst. Die erhaltene Lösung wurde auf das Zellulosepapier gegeben.

Nach 20 min bei Raumtemperatur waren diejenigen Peptidspots, an die der untersuchte Anti-hTSHR-mAb gebunden hatte, aufgrund der Substratreaktion violett angefärbt. Die den angefärbten Spots entsprechenden Peptide wurden auf diese Weise als Bindungsstellen des untersuchten Anti-hTSHR-mAb identifiziert. Die auf diese Weise ermittelten Bindungsstellen der untersuchten Anti-hTSHR-mAb sind in der letzten Spalte der obigen Tabelle 1 aufgeführt. Die Anti-hTSHR-mAb mit den Nummern 9, 10 und 11 banden an keines der vorgelegten Peptïde, was als Hinweis darauf anzusehen ist, daß diese Antikörper 9, 10 und 11 solche sind, die ausschließlich konformationelle Strukturen des rhTSHR erkennen. Die genannten "konformationellen" Anti-hTSHR-mAbs 9, 10 und 11 waren dabei nach dem Verfahren von S. Costagliola und G. Vassart gemäß Veröffentlichung in J. Endocrinol. Invest. 20 (Suppl. 2, No. 5) 1997, Abstract 4 durch Immunisierung von Mäusen mit einem hTSHR-DNA-Konstrukt erhalten worden.

Bei den oben unter 1.4.3 beschriebenen Versuchen zur Bindung des bTSH/rhTSHR-Komplexes mit den verschiedenen Anti-hTSHR-mAb hatte sich gezeigt, wie Tabelle 1 zu entnehmen ist, daß die meisten Antikörper, obwohl in einem erheblichen Überschuß eingesetzt (10 µg Antikörper gegenüber ca. 1 ng rhTSHR!), nicht in der Lage waren, den bTSH/rhTSHR-Komplex vollständig zu präzipitieren. Lediglich die Antikörper 9, 10 und 11 ermöglichten eine vollständige Fällung des genannten bTSH/rhTSHR-Komplexes, und diese erwiesen sich bei der Kartierung als "konformationelle" Antikörper, d.h. solche, die keine bestimmten Peptidesequenzen erkennen, sondern ausschließlich konformationelle Strukturen. Für die Entwicklung eines erfindungsgemäßen Coated-Tube-Assays, bei dem TSHR-Auto-Ab mit Hilfe eines immobilisierten rhTSHR bestimmt werden sollen, wurden daher vorzugsweise die genannten konformationellen Antikörper 9, 10 und 11, die die höchste Bindung lieferten, berücksichtigt.

Es ist jedoch zu betonen, daß die genannten konformationellen Antikörper sich zwar ganz besonders gut zur Immobilisierung von rhTSHR eigneten, daß jedoch auch die restlichen Anti-hTSHR-mAb 1-8 funktionierten, wobei jedoch sehr niedrige Bindungsniveaus erhalten wurden. Derartige monoklonale Antikörper können allein oder gegebenenfalls auch in Form eines Gemischs von zwei oder mehr Anti-hTSHR-mAb zur Bindung von rhTSHR unter Präsentation unterschiedlicher hTSHR-Epitope eingesetzt werden, um im Sinne der Offenbarung in der Patentanmeldung 196 51 093.7 gegebenenfalls zusätzliche Autoantikörper-Subpopulationen im Test zu erfassen und dadurch gegebenenfalls die klinische Wertigkeit zu erhöhen, wenn das sich als erstrebenswert erweisen sollte.

### 1.5 Immobilisieren und Affinitätsreinigen des rhTSHR unter Gewinnung von Teströhrchen für einen Coated-Tube-Assay zur Bestimmung von TSHR-Auto-Ab

Eine krude solubilisierte rhTSHR-Präparation gemäß 1.1 wurde mit 100 mM HEPES; 0,5% Triton X-100; 0,5% Rinderserumalbumin (BSA); pH 7,5% verdünnt, bis unter Verwendung der Reagenzien des kommerziellen TRAK-Assays® der Firma B.R.A.H.M.S Diagnostica GmbH bei der gemäß der Arbeitsvorschrift des genannten Assays durchgeführten PEG-Fällung eine etwa 30%ige Bindung des ¹²⁵I-bTSH erhalten wurde.

Zur Herstellung von beschichteten Röhrchen wurden 200 µl der erhaltenen rhTSHR-Verdünnung mit 100 ng des obigen Anti-hTSHR-mAb Nr. 9 versetzt, und die erhaltene Lösung wurde in Polystyrolröhrchen, deren Wände mit Ziege-anti-Maus-Antikörpern (Maus-IgG-Bindekapazität ca. 100 ng) beschichtet waren, gegeben und bei 4°C 20 h inkubiert.

Die verwendeten Teströhrchen sind solche, wie sie Bestandteil des Kits DYNOtest® TBG der Firma B.R.A.H.M.S Diagnostica GmbH sind.

Nach der Inkubation wurden die Röhrchen mit 2 ml Waschpuffer (10 mM HEPES; 0,1% Triton X-100; pH 7,5) gefüllt, dekantiert und danach in einem Vakuumtrockner für 4 h getrocknet. Durch den Waschschritt wurden unerwünschte Begleitsubstanzen entfernt, so daß die Röhrchen auf ihren Wänden einen hoch affinitätsgereinigten rhTSHR (rhTSHR(imm)*) aufwiesen. Die Röhrchen wurden in die nachfolgend beschriebenen Bestimmungen eingesetzt. Bis zu ihrer Verwendung wurden sie bei 4°C gelagert.

### 2. Pipettier- und Inkubations-Protokolle

Um das Bindungsverhalten von bTSH an das neuartige affinitätsgereinigte rhTSHR-Rezeptorpräparat (rhTSHR(imm)*) genauer zu untersuchen, wurden unter Verwendung der wie oben unter 1.5 beschrieben erhaltenen Teströhrchen mit dem affinitätsgereinigten rhTSHR(imm)* verschiedene Kontrollversuche durchgeführt, wobei eines der folgenden Pipettier-/Inkubations-Protokolle zur Anwendung kam:

### 2.1 Einschritt-Assay

1. Pipettiere die Probe (Patientenserum, Standardserum oder Nullstandardserum; 100 µl) in rhTSHR(imm)* beschichtetes Teströhrchen.
2. Pipettiere dazu ¹²⁵I-bTSH bzw. Akridiniumester-markiertes bTSH, jeweils in 100 µl 100 mM HEPES; 20 mM EDTA; 0,5 mM N-Ethylmaleinimid (NEM); 0,1 mM Leupeptin; 1% BSA; 0,5% Triton X-100; 5 µg Anti-hTSH-Ab (vg1.3.2); pH 7,5).
3. Inkubiere 2 h unter Schütteln (300 U/min) bei Raumtemperatur.
4. Wasche zweimal mit je 2 ml Waschpuffer, dekantiere.
5. Messe die an der Wand des Teströhrchens fixierte Radioaktiviät in einem γ-Counter bzw. löse die Lumineszenzreaktion aus und messe die Chemilumineszenz-Lichtausbeute mit einem geeigneten Luminometer, z.B. Berthold LB 952.

### 2.2 Zweischritt-Assay

1. Pipettiere in ein rhTSHR(imm)*-beschichtetes Teströhrchen Puffer (200 µl; 100 mM HEPES; 20 mM EDTA; 0,5 mM NEM; 0,1 mM Leupeptin; 1% BSA; 0,5% Triton X-100; 5 µg Anti-hTSH-Ab; pH 7,5).
2. Pipettiere die Probe (Patientenserum, Standardserum oder Nullstandardserum; 100 µl).
3. Inkubiere 2 h unter Schütteln (300 U/min) bei Raumtemperatur.
4. Pipettiere 2 ml Waschpuffer (vgl. oben), dekantiere.
5. Pipettiere 200 µl einer markiertes bTSH (¹²⁵I-bTSH oder Akridiniumester-markiertes bTSH) enthaltenden Pufferlösung (10 mM HEPES; 10.000 IU/ml Heparin; 10 mM Calciumchlorid; 1% BSA; 0,1% Triton X-100; pH 7,5).
6. Inkubiere 1 h unter Schütteln (300 U/min) bei Raumtemperatur.
7. Wasche die Röhrchen bei Verwendung von ¹²⁵I-bTSH zweimal mit 2 ml Waschpuffer bzw. bei Verwendung des Akridiniumester-markierten bTSH viermal mit 1 ml Waschpuffer.
8. Messe die an der Wand des Teströhrchens fixierte Radioaktivität in einem γ-Counter bzw. löse die Lumineszenzreaktion aus und messe die Chemilumineszenz-Lichtausbeute mit einem geeigneten Luminometer, z.B. Berthold LB 952.

### 3. Bestimmung von TSHR-Auto-Ab in Patientenseren

### 3.1 Zweischritt-Assay zur Bestimmung von TSHR-Auto-Ab und seine Vorteile

Durch verschiedene Kontrollversuchen abgesicherte Erkenntnisse ermöglichen es, die TSHR-Auto-Ab-Bestimmung unter Verwendung von rhTSHR(imm)*-beschichteten Teströhrchen als Zweischritt-Assay durchzuführen, bei dem im zweiten Schritt ein serumfreies und daher gut standardisierbares markiertes bTSH-Präparat eingesetzt wird. Der Einsatz von markiertem bTSH in einem zweiten Assay-Schritt in Abwesenheit aller nicht an den immobilisierten rhTSHR gebundenen Probenbestandteile schließt sicher eine Meßwertverfälschung durch Anti-bTSH-Ab aus, die in signifikantem Ausmaße in verschiedenen Patientenseren vorkommen (vgl. obige Erläuterung zu Anti-bTSH-Ab).

Derartige Anti-bTSH-Ab in Patientenseren führen gemäß dem herkömmlichen Assay-Protokoll zu "falsch" erniedrigten Meßwerten für die TSHR-auto-Ab-Konzentration, da derartige Antikörper zusammen mit dem gebundenen markierten bTSH bei der PEG-Fällung mit ausgefällt werden und eine zu niedrige Bindung von TSHR-Auto-Ab vortäuschen.

Bei der Messung nach dem o.g. Einschritt-Assay-Protokoll führt die Anwesenheit von Anti-bTSH-Ab zu "falsch" erhöhten Meßwerten, da zuwenig markiertes bTSH an die Wände des Teströhrchens gebunden wird.

Im Zweischritt-Verfahren kommt es zu keinem Kontakt zwischen der Probe und dem markierten bTSH, so daß Anti-bTSH-Ab keinen Störfaktor mehr darstellen, und zwar insbesondere dann, wenn im zweiten Schritt serumfrei gearbeitet wird. Derzeit wird daher die Zweischritt-Variante für die Durchführung von TSHR-Auto-Ab bevorzugt.

### 3.2 Verhinderung einer Meßverfälschung durch pathologisch erhöhte hTSH-Konzentrationen in Patientenseren.

Aufgrund der Verwendung eines humanen TSHR-Präparats als Rezeptorbinder war zu prüfen, ob die Ergebnisse der Bestimmungen von TSHR-Auto-Ab unter Verwendung der o.g. beschichteten Teströhrchen durch die Anwesenheit von hTSH in Patientenproben gestört werden kann. hTSH kommt normalerweise in Humanseren in außerordentlich geringen, nicht störenden Konzentrationen vor. Es ist jedoch bekannt, daß im hypothyreoten Zustand pathologisch erhöhte hTSH-Konzentrationen vorkommen können.

Ein von hTSH befreites Serum (Skandibodies, USA) wurde zur Gewinnung definierter Probenlösungen mit steigenden Mengen an hTSH (Boehringer Mannheim, Deutschland) versetzt. Die erhaltenen Seren wurden als Proben nach dem obigen Zweischritt-Assay-Protokoll (2.2) vermessen.

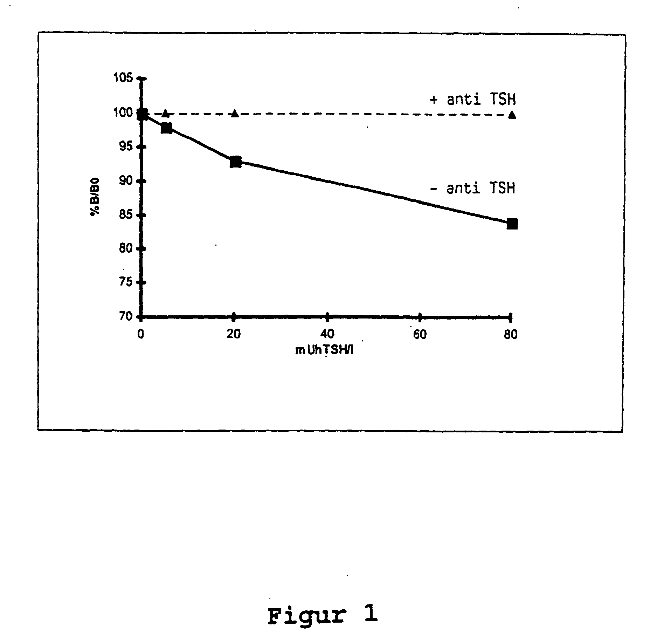

Wie Fig. 1 zeigt, kommt es bei pathologisch hohen hTSH-Konzentrationen zu einer Beeinflussung der Bindung des markierten bTSH durch Kompetition mit dem hTSH.

Indem man der Probenlösung einen hTSH-Antikörper zusetzt, wird der Einfluß der Anwesenheit von hTSH in der Probe aufgehoben. Ein geeigneter hTSH-Antikörper ist der kommerziell erhältliche Antikörper mit der Artikel-Nr. 5404 der Firma Oy Medix Biochemica Ab, Finnland. Durch Zusatz eines Überschusses eines derartigen Antikörpers (z.B. 5 µg/Test) wird der Einfluß von erhöhten hTSH-Konzentrationen beseitigt. Da der genannte hTSH-Antikörper keine Kreuzreaktion mit bTSH zeigt, kann er der Probenlösung auch bei der Einschritt-Assay-Variante (vgl. 2.1, Schritt 2.) zugesetzt werden und führt zur gleichen Verbesserung der Meßergebnisse.

### 3.3 Verbesserter Assay zur TSHR-Auto-Ab Bestimmung und seine Prüfung in der Praxis

Unter Berücksichtigung aller obigen Erkenntnisse wurde somit ein neuartiger Assay zur Bestimmung von TSHR-Auto-Ab in Patientenseren bzw. -plasmen erhalten, dessen Zuverlässigkeit durch Vermessen eines Kollektivs von Patientenseren überprüft werden konnte, wobei das Zweischritt-Assay-Inkubationsprotokoll (2.2) zur Anwendung kam.

### 3.3.1 Standardkurven

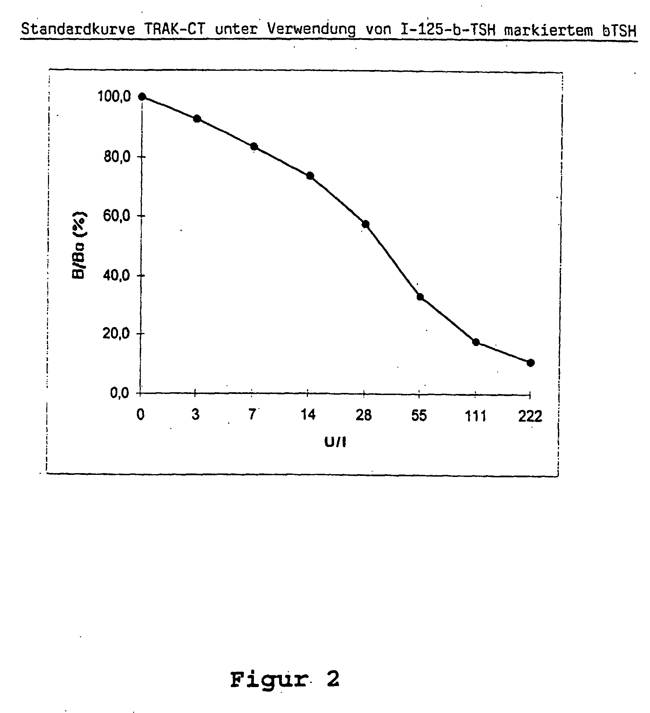

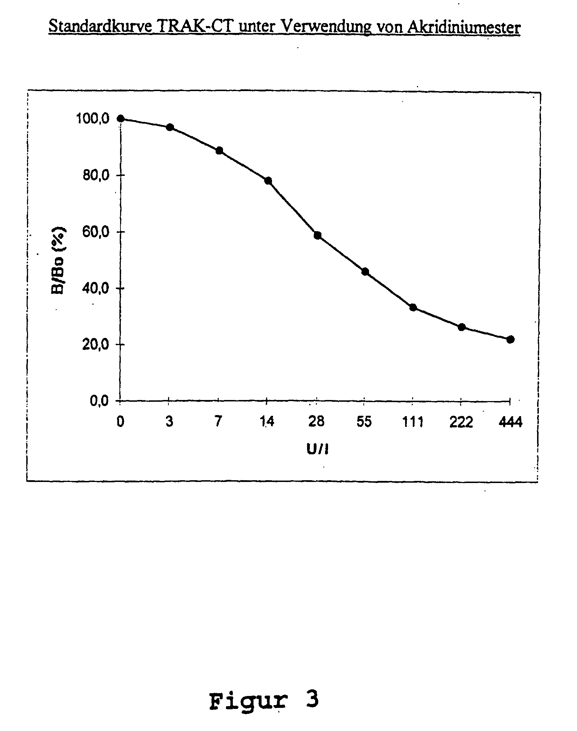

Die Figuren 2 und 3 zeigen die unter Verwendung der Standards gemäß 1.3 erhaltenen Standardkurven bei Verwendung einmal von ¹²⁵I-bTSH (Fig.2) bzw. von Akridiniumester-markiertem bTSH (Fig.3). Der Nullstandard ist dabei ein Poolserum von Schilddrüsen-gesunden Menschen; Standards 400 bis 3 sind Mischungen aus Poolseren von Schilddrüsen-gesunden und von Morbus Basedow-Patienten. Die Kalibrierung erfolgte unter Verwendung des herkömmlichen TRAK-Assay® der Firma B.R.A.H.M.S Diagnostica.

### 3.3.2 Messung von Patientenseren

Unabhängig von der Art der Markierung des bTSH reduzieren TSHR-Auto-Ab die Bindung des markierten bTSH an die Oberfläche der Teströhrchen in konzentrationsabhängiger Weise, wobei schon ab 3 U/l ein deutlich positives Signal feststellbar war.

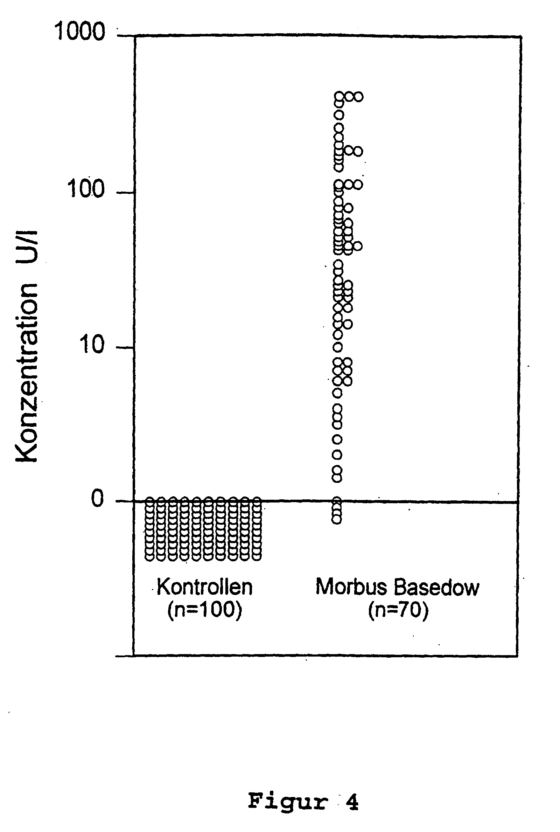

Nach dem beschriebenen Zweischritt-Assay-Protokoll (2.1.2) wurden 100 Kontrollseren Schilddrüsen-gesunder und 70 Seren von Morbus Basedow-Patienten vermessen. Die Ergebnisse sind in Fig. 4 gezeigt. Es ist deutlich die starke Differenzierung zwischen Seren von Patienten mit Morbus Basedow, die TSHR-Auto-Ab enthalten, und Schilddrüsen-gesunden Kontrollpersonen zu erkennen.

Die Erfindung ermöglicht eine Verbesserung von Verfahren zur Bestimmung von TSHR-Auto-Ab sowohl im Hinblick auf einen Ausschluß von physiologischen Störfaktoren, die die Meßergebnisse in bisherigen Assays verfälschen konnten, als auch insbesondere in praktischer Hinsicht. Gestützt auf die Lehre in der vorliegenden Anmeldung ist es erstmals möglich, alle oder wenigstens die meisten Reagenzien für die Durchführung eines solchen Verfahrens in gebrauchsfertiger Form herzustellen und zu Anwendern zur Verfüung zu stellen (z.B. in Form vorbeschichteter Teströhrchen und standardisierter, serumfreier Markierungsreagenzien).

## Patentansprüche

1. Verfahren zur Bestimmung von TSH-Rezeptor-Autoantikörpern (TSHR-Auto-Ab) in einer biologischen Probe mit Hilfe eines Rezptorbindungsassays, bei dem man die Probe mit (i) einem TSH-Rezeptor-Präparat (TSHR-Präparat) und (ii) markiertem bovinem TSH (bTSH) umsetzt, und bei dem man die Anwesenheit und/oder Menge der zu bestimmenden TSHR-Auto-Ab in der biologischen Probe auf der Basis der gebundenen Menge an markiertem bTSH in dem von der flüssigen Phase abgetrennten Komplex aus bTSH bzw. TSHR-Auto-Ab mit dem TSHR ermittelt,
**dadurch gekennzeichnet, daß** man als TSHR-Präparat einen funktionalen rekombinanten TSH-Rezeptor (rTSHR) verwendet, der mit Hilfe eines selektiven Antikörpers gegen den TSH-Rezeptor (Anti-TSHR-Ab) an eine feste Phase gebunden, im gebundenen Zustand durch Waschen gereinigt und auf diese Weise in einen affinitätsgereinigten immobilisierten rTSHR (rTSHR (imm)*) überführt wurde.

2. Verfahren nach Anspruch 1, **dadurch gekennzeichnet, daß** man als funktionalen rekombinanten TSH-Rezeptor einen funktionalen rekombinanten humanen TSH-Rezeptor (rhTSHR) verwendet und als selektiven Antikörper wenigstens einen monoklonalen Antikörper gegen den humanen TSH-Rezeptor (Anti-hTSHR-mAb) verwendet.

3. Verfahren nach Anspruch 1 oder 2, **dadurch gekennzeichnet, daß** man als feste Phase die Wände von Teströhrchen verwendet, die mit einem tierspezifischen Antikörper zur Bindung des Anti-TSHR-Ab vorbeschichtet sind.

4. Verfahren nach einem der Ansprüche 2 oder 3, **dadurch gekennzeichnet, daß** wenigstens einer der monoklonalen Antikörper ein monoklonaler Antikörper gegen den humanen TSH-Rezeptor (Anti-hTSHR-mAb) ist, der nur konformationelle Strukturen des rhTSHR erkennt, wie er nach der Technik der Immunisierung mit einem DNA-Konstrukt erhältlich ist.

5. Verfahren nach Anspruch 1, 2, 3 oder 4, **dadurch gekennzeichnet, daß** man den Rezeptorbindungsassay als Einschritt-Assay in einem mit einem affinitätsgereinigten rhTSHR(imm)* beschichteten Teströhrchen durchführt, bei dem man durch Pipettieren (i) der serumhaltigen Probe und (ii) einer Pufferlösung, die das markierte bTSH-Präparat enthält, eine einzige Reaktionslösung herstellt, diese nach einer Inkubation für einen ausreichenden Zeitraum aus dem Teströhrchen entfernt, das Teströhrchen wäscht und die an die Wände des Teströhrchens gebundene Markierung auf an sich bekannte Weise mißt.

6. Verfahren nach Anspruch 1, 2, 3 oder 4, **dadurch gekennzeichnet, daß** man den Rezeptorbindungsassay als Zweischritt-Assay in einem mit einem affinitätsgereinigten rhTSHR(imm)* beschichteten Teströhrchen durchführt, bei dem man (i) im ersten Schritt nur die Probe und einen Puffer pipettiert, inkubiert, danach dekantiert und die Teströhrchen wäscht und (ii) im zweiten Schritt eine Pufferlösung, die das markierte bTSH-Präparat enthält, in das Teströhrchen gibt, nach einer Inkubation für einen ausreichenden Zeitraum die flüssige Phase aus dem Teströhrchen entfernt, das Teströhrchen wäscht und die an die Wände des Teströhrchen gebundene Markierung auf an sich bekannte Weise mißt.

7. Verfahren nach Anspruch 1 oder 2, **dadurch gekennzeichnet, daß** man es in automatisierter Form durchführt, wobei man als Festphase suspendierte Partikel verwendet, die mit einem selektiven Anti-TSHR-Ab beschichtet sind, und daß man die Präparation des rTSHR und die Probe so zusetzt, daß zeitweise eine beide genannten Assaykomponenten enthaltende Probenlösung gebildet wird.

8. Verfahren nach Anspruch 6, **dadurch gekennzeichnet, daß** man das markierte bTSH im zweiten Schritt in einer serumfreien Pufferlösung zugibt.

9. Verfahren nach einem der Ansprüche 1 bis 8, **dadurch gekennzeichnet, daß** man die Umsetzung der Probe mit dem affinitätsgereinigten rhTSHR(imm)* in Gegenwart mindestens eines Antikörpers gegen humanes TSH (Anti-hTSH-Ab) durchführt.

10. Verfahren nach Anspruch 9 in Rückbeziehung auf Anspruch 5, **dadurch gekennzeichnet, daß** der mindestens eine Antikörper gegen humanes TSH so ausgewählt ist, daß er nicht mit bovinem TSH kreuzreagiert.

11. Verfahren nach einem der Ansprüche 1 bis 10, **dadurch gekennzeichnet, daß** die zu bestimmenden TSH-Rezeptor-Autoantikörper rezeptorstimulierende Autoantikörper sind, deren Auftreten in einem Humanserum für den Morbus Basedow charakteristisch ist.

12. Verfahren zur Bestimmung von TSH-Rezeptor-Autoantikörpern (TSHR-Auto-Ab) in einer biologischen Probe mit Hilfe eines Rezptorbindungsassays, bei dem man die Probe mit (i) einem TSH-Rezeptor-Präparat (TSHR-Präparat) und (ii) markiertem bovinem TSH (bTSH) umsetzt, und bei dem man die Anwesenheit und/oder Menge der zu bestimmenden TSHR-Auto-Ab in der biologischen Probe auf der Basis der gebundenen Menge an markiertem bTSH in dem von der flüssigen Phase abgetrennten Komplex aus bTSH bzw. TSHR-Auto-Ab ermittelt,
**dadurch gekennzeichnet, daß** man den Rezeptorbindungsassay als Zweischritt-Assay in einem Teströhrchen durchführt, das mit einem affinitätsgereinigten rhTSHR(imm)* beschichtet ist, bei dem man (i) im ersten Schritt eine Probe, die einen Zusatz eines Anti-hTSH-Antikörpers enthält, und einen Puffer pipettiert, inkubiert, danach die flüssige Phase aus dem Teströhrchen entfernt und die Teströhrchen wäscht und (ii) im zweiten Schritt eine serumfreie Pufferlösung, die das markierte bTSH-Präparat enthält, in das Teströhrchen gibt, nach einer Inkubation für einen ausreichenden Zeitraum die flüssige Phase aus dem Teströhrchen entfernt, das Teströhrchen wäscht und die an die Wände des Teströhrchen gebundene Markierung auf an sich bekannte Weise mißt.

13. Reagenziensatz zur Durchführung eines kompetitiven Rezeptorbindungsassays nach einem der Ansprüche 1 bis 12, **dadurch gekennzeichnet, daß** er, neben weiteren üblichen Komponenten eines derartigen Reagenziensatzes, wenigstens enthält:
(i) mit einem rhTSHR in Form eines affinitätsgereinigten immobilisierten rhTSHR beschichtete Teströhrchen,
(ii) markiertes TSH oder einen markierten spezifischen TSH-Rezeptor-Antikörper in einer serumfreien Pufferlösung.

## Claims

1. Method for the determination of TSH receptor autoantibodies (TSHR-auto-ab) in a biological sample with the aid of a receptor binding assay, in which the sample is reacted with (i) a TSH receptor preparation (TSHR preparation) and (ii) labelled bovine TSH (bTSH) and in which the presence and/or amount of the TSHR-auto-ab to be determined in the biological sample is determined on the basis of the bound amount of labelled bTSH in the complex, separated from the liquid phase, of bTSH or TSHR-auto-ab with the TSHR, **characterized in that** the TSHR preparation used is a functional recombinant TSH receptor (rTSHR) which is bound to a solid phase with the aid of a selective antibody against the TSH receptor (anti-TSHR-ab), purified in the bound state by washing and converted in this manner into an affinity-purified immobilized rTSHR (rTSHR(imm)*.

2. Method according to Claim 1, **characterized in that** the functional recombinant TSH receptor used is a functional recombinant human TSH receptor (rhTSHR) and the selective antibody used is at least one monoclonal antibody against the human TSH receptor (anti-hTSHR-mab).

3. Method according to Claim 1 or 2, **characterized in that** the solid phase used comprises the walls of test tubes which are precoated with an animal-specific antibody for binding the anti-TSHR-ab.

4. Method according to either of Claims 2 and 3, **characterized in that** at least one of the monoclonal antibodies is a monoclonal antibody against the human TSH receptor (anti-hTSHR-mab), which recognizes only conformational structures of the rhTSHR, as obtainable by the technique of immunization with a DNA construct.

5. Method according to Claim 1, 2, 3 or 4, **characterized in that** the receptor binding assay is carried out as a one-step assay in a test tube coated with an affinity-purified rhTSHR(imm)*, in which assay a single reaction solution is prepared by pipetting (i) the serum-containing sample and (ii) a buffer solution which contains the labelled bTSH preparation, said reaction solution is removed from the test tube after incubation for a sufficient period, the test tube is washed and the tracer bound to the walls of the test tube is measured in a manner known per se.

6. Method according to Claim 1, 2, 3 or 4, **characterized in that** the receptor binding assay is carried out as a two-step assay in a test tube coated with an affinity-purified rhTSHR(imm)*, in which assay, (i) in the first step, only the sample and a buffer are pipetted, incubated and then decanted and the test tubes are washed and, (ii) in the second step, a buffer solution which contains the labelled bTSH preparation is added to the test tube, the liquid phase is removed from the test tube after incubation for a sufficient period, the test tube is washed and the tracer bound to the walls of the test tube is measured in a manner known per se.

7. Method according to Claim 1 or 2, **characterized in that** it is carried out in automated form, the solid phase used comprising suspended particles which are coated with a selective anti-TSHR-ab, and that the preparation of the rTSHR and the sample are added in such a way that a sample solution containing said two assay components is temporarily formed.

8. Method according to Claim 6, **characterized in that** the labelled bTSH is added in the second step in a serum-free buffer solution.

9. Method according to any of Claims 1 to 8, **characterized in that** the reaction of the sample with the affinity-purified rhTSHR(imm)* is carried out in the presence of at least one antibody against human TSH (anti-hTSH-ab).

10. Method according to Claim 9, relating back to Claim 5, **characterized in that** the at least one antibody against human TSH is chosen so that it does not cross-react with bovine TSH.

11. Method according to any of Claims 1 to 10, **characterized in that** the TSH receptor autoantibodies to be determined are receptor-stimulating autoantibodies whose occurrence in a human serum is characteristic of Graves' disease.

12. Method for the determination of TSH receptor autoantibodies (TSHR-auto-ab) in a biological sample with the aid of a receptor binding assay, in which the sample is reacted with (i) a TSH receptor preparation (TSHR preparation) and (ii) labelled bovine TSH (bTSH) and in which the presence and/or amount of the TSHR-auto-ab to be determined in the biological sample is determined on the basis of the bound amount of labelled bTSH in the complex, separated from the liquid phase, of bTSH or TSHR-auto-ab, **characterized in that** the receptor binding assay is carried out as a two-step assay in a test tube which is coated with an affinity-purified rhTSHR(imm)*, in which assay, (i) in the first step, a sample, which contains an added anti-hTSH antibody and a buffer are pipetted and incubated, the liquid phase is then removed from the test tube and the test tubes are washed and, (ii) in the second step, a serum-free buffer solution which contains the labelled bTSH preparation is added to the test tube, the liquid phase is removed from the test tube after incubation for a sufficient period, the test tube is washed and the tracer bound to the walls of the test tube is measured in a manner known per se.

13. Reagent kit for carrying out a competitive receptor binding assay according to any of Claims 1 to 12, **characterized in that**, in addition to further conventional components of a reagent kit of this type, it contains at least:
(i) test tubes coated with a rhTSHR in the form of an affinity-purified immobilized rhTSHR,
(ii) labelled TSH or a labelled specific TSH receptor antibody in a serum-free buffer solution.

## Revendications

1. Procédé pour la détermination d'auto-anticorps des récepteurs de TSH ("TSHR-auto-Ab") dans un échantillon biologique, à l'aide d'une détermination de liaison à un récepteur, dans lequel on fait réagir l'échantillon avec (i) une préparation de récepteur de TSH (préparation TSHR) et (ii) du TSH bovin marqué (bTSH), et dans lequel on détermine la présence et/ou la quantité de TSHR-auto-Ab à déterminer dans l'échantillon biologique, sur base de la quantité liée de bTSH marqué dans le complexe constitué de bTSH et de TSHR-auto-Ab séparé de la phase liquide,
**caractérisé en ce que**, comme préparation de TSHR, on utilise un récepteur de TSH fonctionnel recombiné (rTSHR) qui est lié à une phase solide à l'aide d'un anticorps sélectif dirigé contre le récepteur TSH (anti-TSHR-Ab), que l'on nettoie par lavage à l'état lié et que l'on transfère de cette manière dans un rTSHR immobilisé (rTSHR(imm)') purifié par affinité.

2. Procédé selon la revendication 1, **caractérisé en ce que** l'on utilise comme récepteur de TSH fonctionnel recombiné un récepteur de TSH fonctionnel recombiné humain (rhTSHR) et que l'on utilise comme anticorps sélectif au moins un anticorps monoclonal dirigé contre le récepteur de TSH humain (anti-hTSHR-mAb).

3. Procédé selon la revendication 1 ou 2, **caractérisé en ce que** comme phase solide, on utilise les parois de tube à essai qui sont préalablement enduites d'un anticorps animal qui se lie spécifiquement à l'anti-TSHR-Ab.

4. Procédé selon l'une des revendications 2 ou 3, **caractérisé en ce qu'**au moins l'un des anticorps monoclonaux est un anticorps monoclonal dirigé contre le récepteur de TSH humain (anti-hTSHR-mAb) qui ne reconnaît que les structures de conformation du rhTSHR et qui a été obtenu par la technique d'immunisation par un construct d'ADN.

5. Procédé selon les revendications 1, 2, 3 ou 4, **caractérisé en ce que** l'on réalise la détermination par liaison à récepteur comme détermination à une étape dans un tube à essai revêtu de rhTSHR(imm)' purifié par affinité, dans lequel on prépare une unique solution de réaction par pipettage (i) de l'échantillon contenant du sérum et (ii) d'une solution tampon qui contient la préparation de bTSH marqué, que l'on enlève cette solution de réaction du tube à essai après une incubation pendant une durée suffisante, que l'on lave le tube à essai et que l'on mesure de manière connue en soi le marqueur lié aux parois du tube à essai.

6. Procédé selon la revendication 1, 2, 3 ou 4, **caractérisé en ce que** l'on réalise la détermination par liaison à récepteur comme détermination en deux étapes dans un tube à essai revêtu de rhTSHR(imm)' purifié par affinité, dans lequel on pipette (i) dans la première étape uniquement l'échantillon et un tampon, que l'on incube, ensuite que l'on décante et que l'on lave les tubes à essais, et (ii), dans une deuxième étape, on introduit dans le tube à essai une solution tampon qui contient la préparation de bTSH marqué, on enlève la phase liquide du tube à essai après incubation pendant une durée suffisante, on lave le tube à essai et on mesure de manière connue en soi le marqueur lié aux parois du tube à essai.

7. Procédé selon la revendication 1 ou 2, **caractérisé en ce qu'**on l'exécute sous forme automatisée, en utilisant comme phase solide des particules en suspension qui sont revêtues d'un anti-TSHR-Ab sélectif, et **en ce que** l'on ajoute la préparation de rTSHR et de l'échantillon de manière à former temporairement une solution d'échantillon contenant lesdits deux composants de détermination.

8. Procédé selon la revendication 6, **caractérisé en ce que** dans la deuxième étape, on ajoute le bTSH marqué dans une solution tampon exempte de sérum.

9. Procédé selon l'une des revendications 1 à 8, **caractérisé en ce que** la réaction de l'échantillon avec le rhTSHR(imm)' purifié par affinité est réalisée en présence d'au moins un anticorps dirigé contre le TSH humain (anti-hTSH-Ab).

10. Procédé selon la revendication 9 et selon la revendication 5, **caractérisé en ce que** l'anticorps au moins prévu, dirigé contre le TSH humain, est sélectionné de telle sorte qu'il n'entre pas en réaction croisée avec le TSH de bovin.

11. Procédé selon l'une des revendications 1 à 10, **caractérisé en ce que** les anticorps du récepteur de TSH à déterminer sont des auto-anticorps stimulant les récepteurs et dont la présence dans un sérum humain est caractéristique de la maladie de Basedow.

12. Procédé pour la détermination d'auto-anticorps des récepteurs de TSH (TSHR-auto-Ab) dans un échantillon biologique à l'aide d'une détermination par liaison à récepteur, dans lequel on fait réagir l'échantillon avec (i) une préparation de récepteur de TSH (préparation de TSHR) et (ii) du TSH bovin marqué (bTSH), et dans lequel on détermine la présence et/ou la quantité du TSHR-auto-Ab à déterminer dans l'échantillon biologique sur base de la quantité liée de bTSH marqué dans le complexe constitué de bTSH et TSHR-auto-Ab séparé de la phase liquide, **caractérisé en ce que** l'on réalise la détermination par liaison à récepteur comme détermination en deux étapes dans un tube à essai qui a été revêtu d'un rhTSHR(imm)' purifié par affinité, dans lequel (i) dans une première étape, on pipette un échantillon qui. comprend une addition d'un anticorps anti-hTSH et un tampon, on incube, on sépare ensuite la phase liquide du tube à essai et on lave le tube à essai, et (ii) dans une deuxième étape, on introduit dans le tube à essai une solution tampon exempte de sérum et contenant la préparation de bTSH marqué, on sépare la phase liquide du tube à essai après une incubation d'une durée suffisante, on lave le tube à essai et on mesure de manière connue le marqueur lié aux parois du tube à essai.

13. Trousse de réactif pour la réalisation d'une détermination par liaison à récepteur selon l'une des revendications 1 à 12, **caractérisée en ce qu'**en plus d'autres composants habituels, une telle trousse de réactifs contient au moins:
(i) des tubes à essai revêtus d'un rhTSHR sous la forme d'un rhTSHR immobilisé et purifié par affinité, et
(ii) du TSH marqué ou un anticorps marqué, spécifique du récepteur de TSH, dans une solution tampon exempte de sérum.
